# EUROPEAN PATENT SPECIFICATION

(11) **EP 2 718 690 B1**
(45) Date of publication and mention of the grant of the patent: **03.01.2018**
(21) Application number: 12797143.0
(22) Date of filing: 08.06.2012
(51) Int. Cl.: G01N 15/14, G01N 33/483, C12Q 1/02, G01N 21/00, B01L 3/00

(54) **METHODS AND APPARATUS FOR IMPROVING IN VITRO MEASUREMENTS USING BOYDEN CHAMBERS**
VERFAHREN UND VORRICHTUNG ZUR VERBESSERUNG VON IN-VITRO-MESSUNGEN MIT BOYDEN-KAMMERN
PROCÉDÉS ET APPAREIL POUR AMÉLIORER DES MESURES IN VITRO UTILISANT DES CHAMBRES DE BOYDEN

(30) Priority: 10.06.2011 US 201113157873
(43) Date of publication of application: 16.04.2014
(73) Proprietor: Essen Instruments, Inc., Ann Arbor, MI 48108 (US)
(72) Inventor: SCHROEDER, Kirk, Carmel, CA 93921 (US); NEAGLE, Bradley D., Ann Arbor, MI 48108 (US)
(74) Representative: Pallini Gervasi, Diego
(86) International application number: PCT/US2012/041652
(87) International publication number: WO 2012/170880

(56) References cited:
- WO-A1-00/55298
- WO-A1-00/55298
- WO-A1-01/32827
- WO-A2-2007/106868
- US-A1- 2002 185 384
- US-A1- 2005 164 404

## Description

### FIELD OF THE INVENTION

This invention relates generally to a biological measurement apparatus and a biological measurement method.

### BACKGROUND OF THE INVENTION

Cell migration is critical in many physiological processes. Chemotaxis, for example, is the study of cell motion in response to a soluble chemo-attractant stimulus. Similar and related mechanisms include haptotaxis and chemoinvasion, which rely on cell motility on a substrate-bound stimulus and movement through an extracellular matrix (ECM) boundary layer, respectively. These processes play a vital role in the basic physiology of many diseases in a large number of therapeutic areas and the study of cell migration is a widely adopted research tool for both *in vitro* and *in vivo* biological research.

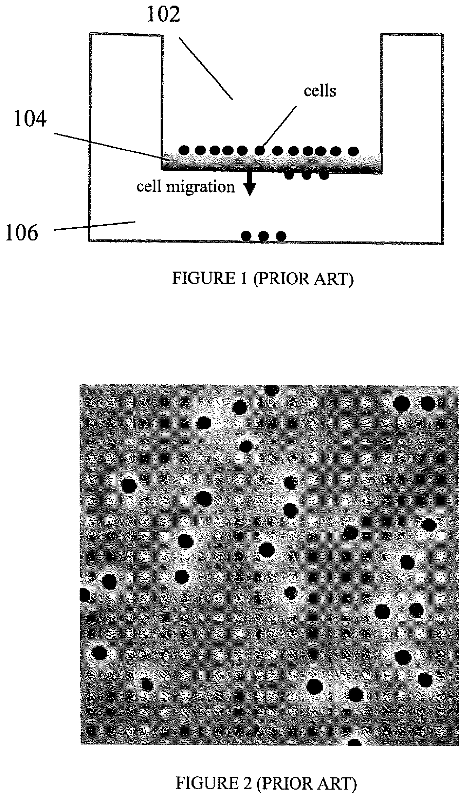

The most common technique for measuring cell migration *in vitro* is via a measurement geometry known as the Boyden chamber [1] first described in 1962. This geometry, developed by Dr. Stephen Boyden, consists of two chambers separated by a porous membrane. Figure 1 illustrates a prior-art Boyden Chamber single-well geometry for measuring cell migration utilizing a porous track-etch membrane filter 104. Migrating cells respond to a chemical gradient that forms by diffusion of the chemoattractant from the lower chamber 106 to the upper chamber 102 via the porous membrane 104. Cells respond to the chemical gradient by directional cues, migrating through the holes of the porous membrane. Cells which migrate through the holes of the membrane can either adhere to the lower side of the membrane, or fall through the membrane to a lower reservoir for detection.

For biological applications, the membrane pores are typically in the range of 3 to 8 uM in diameter, slightly smaller than the diameter of most cells, but large enough that cells can squeeze through. When using a Boyden chamber as a chemotaxis measuring device, a chemoattractant is typically added to the lower chamber, cells are added to the upper chamber and the porous membrane serves as a means to establish a diffusion-based, time-dependent chemical gradient between the upper and lower chambers [2]. The cells on the top side of the membrane detect the chemical gradient, migrate to the individual pores in the membrane, and then crawl through the holes to the lower chamber. After migrating through the small pores, the cells ultimately either fall through the membrane to a lower reservoir, or remain attached on the bottom side of the membrane.

As an *in vitro* assay for chemotactic cell migration, test compounds (drugs) are either added to the upper chamber at the start of the experiment, or the cells are pre-incubated with the compound prior to loading the Boyden chamber device. Pharmacological modulation is then measured by comparing the migration response in cells exposed to test compound vs. cells with no test compound. Quite often in using these devices there is also a correction made for cell migration which may occur in the measurement chambers which contain no chemoattractant in the lower reservoir. This "random migration" component can then be subtracted off the response in order to deduce the number of cells responding to the chemotactic gradient alone.

Determination of the chemotactic response relies on quantifying the number of cells that have migrated through the porous membrane. Historically, this is done by fluorescently labeling the cells either prior to adding to the Boyden chamber (pre-labeling), or after the cells have migrated (post labeling). The fluorescence detection is then performed using an imaging system (fluorescence microscopy) and counting the individual fluorescently labeled cells which have migrated, or by making a bulk fluorescence measurement with a fluorescent plate reader and subsequently calibrating this bulk fluorescent signal to total cell number.

There have been many improvements to the standard Boyden chamber geometry since its inception, but the basic device geometry and membrane components have remained fairly consistent. Goodwin, in a series of U.S. patents (Nos. 5,210,021; 5,284,753 and 5,302,515) describes improvements wherein a device is constructed of multiple sites, often using hydrophobic coatings to make the individual well compartments. While extending the art at the time of invention, these devices all rely on labeling the cells with a fluorescent dye for detection. Another disadvantage of these inventions is the requirement to scrape away cells on the top side of the membrane in order to differentiate them from those which have migrated to the bottom side of the membrane.

In U.S. Patent 5,601,997, Tchao describes a device to help eliminate this problem by using an organic absorbtion dye in the porous membrane to block fluorescent excitation light from getting to the top side of the membrane. In this way, fluorescent pre-labeled cells which had migrated to the bottom side of the membrane, or migrated through the membrane and fallen into a lower reservoir could be counted using a fluorescent reader without fluorescent signal interference from the cells remaining on the top side of the membrane. This invention extended the state-of-the art by not requiring the tedious and error-prone cell "scraping" steps. It also enabled the possibility of performing kinetic, time-lapse experiments using fluorescent pre-labeled cells.
Furthermore, the international patent application WO 00/55298 A1 discloses a cell activity assay apparatus and method which uses electromagnetic radiation detection beams to measure cell activity. The apparatus comprises an opaque membrane with a plurality of off-axis pores in order to prevent significant amounts of the radiation from traversing the membrane.

Currently, there are many commercial sources for 6-well, 24-well or 96-well Boyden chamber-derived chemotaxis kits, including the ChemoTx ™ system sold by Neuroprobe Inc. (Gaithersburg, MD), the Transwell ™ system sold by Corning Life Science (Acton, MA) and the HTS Fluoroblo^{k} ™ system sold by Becton-Dickinson (Franklin Lakes, NJ). All of these devices are basically rectangular arrays of Boyden chambers using standard microplate formats and injection mold fabrication techniques for creating upper and lower reservoirs separated by a porous filter membrane. All of these commercial devices also use the same basic material for the porous membrane which is known as a "track-etch" membrane.

Track-etch membranes are manufactured by exposing 10 to 20 micron thick, polymer films (e.g. polyester, or polycarbonate) to radioactive particle bombardment, followed by chemical etching [3]. The results of this manufacturing process are a porous film with a random pattern of precisely sized micro-holes as shown in the high-resolution microscopic brightfield image of Figure 2. The cumulative density of micro-holes using this fabrication technique is controlled by the exposure time to, as well as the physical geometry between, the membrane and the radioactive source. The size of the micro-holes in a given track-etch membrane is governed by a combination of time, temperature and the chemical concentration used during the etch step. Typical etch solutions include highly concentrated NaOH, or HF. For a given process, the micro-hole size is very uniform, and in general the holes are orthogonal to the surface of the membrane. Pore size typically ranges from 0.2 microns, up to 10 microns in diameter. As shown in Figure 2, the pores are in random locations with some certain local areas on the membrane having much higher pore densities than other areas on the membrane. The random nature of the radioactive bombardment does not allow for precise control of the local pore spacing or density.

The predominant application for track-etched membranes is fine particle and contaminant filtering of fluids as well a method of capturing and detecting microorganisms. The filtering applications take advantage of the very uniform and defined pore size of the membrane. This characteristic makes these types of membranes ideal for precisely filtering particles or micro-organisms of a given size. For biological applications such as cell migration, track-etch membranes of 2, 3, 5 and 8 micron diameter pore sizes are most commonly used with pore densities ranging from 1000 pores per square millimeter to 20,000 pores per square millimeter.

For *in vitro* chemotactic cell migration applications, pore size is often matched to size of the cells being studied, bigger cells use bigger pore sizes. Typically, the pore size is chosen to be slightly larger than the nucleus of the cells under investigation. Most of the commercial manufacturers of Boyden-style chemotaxis chambers offer a variety of products incorporating different pore sizes. They also offer membranes in different materials, most commonly polycarbonate and polyester, and often supply biological substrate coatings (e.g. collagen, fibronectin or laminin) or surface coating protocols. These biological coatings are sometimes useful so as to more accurately mimic the *in vivo* surface/adhesion biology of migrating cells and often the surface coatings are necessary to illicit the proper surface receptor activation (e.g. integrin signaling) necessary for the cells to migrate efficiently [4].

The quantitative read-out from a Boyden chamber chemotaxis assay is based on quantifying the number of cells which migrate through the individual holes of the track-etch membrane. The cells which crawl through the pores, either adhere onto the bottom side of the membrane, or alternatively fall into the lower reservoir. In such test systems it is often necessary to include "control wells" which do not contain chemoattractant in the bottom reservoir to correct for the occurrence of random (non-directed) migration which can occur. In existing commercial Boyden chamber technologies, quantification of the number of cells is accomplished by using fluorescent dye labeling of the cells.

Labeling of the cells is necessary as the cells cannot be visualized on the surface of the track-etch membranes directly without using a labeling dye. Today, there are a host of live-cell dye markers which can be used, such as Calcein-AM (Sigma Aldrich, St. Louis MO.). Once labeled, the cells can be counted directly using cell counting microscopy; or if a proportionality relationship can be established between fluorescence and cell number, a bulk fluorescent measurement can be made as a surrogate for cell number. Cells are typically labeled at the beginning of the experiment, i.e. before cell migration occurs. Cells can also be "post-labeled," i.e. after the cell migration occurs. This latter method is often preferred when working with cell types which are adversely affected by the fluorescent dyes.

Boyden chamber technology is the current "gold standard" for *in vitro* chemotaxis and chemoinvasion type assays and has been around for almost fifty years. The modern incarnations of the technique have the advantage of being amenable to multi-well microplate formats and the precision of plastic injection molding techniques; as such they are reasonably high throughput assays. While being the current gold standard, and clearly dominating the research market, there are several disadvantages to the current Boyden chamber systems. These disadvantages will be discussed in the following paragraphs.

### DISADVANTAGES OF PRIOR ART BOYDEN CHAMBER GEOMETRIES

### A. Current Bovden Chamber devices are not compatible with phase-contrast imaging

One limitation of the current Boyden chamber technologies is the inability to image the cells on the surface of the membrane *in-situ,* **without** using fluorescent labels. The reason for this has to do with the optical quality of the track-etch membranes used in commercial Boyden chamber products. Biological cells are essentially optically transparent. Imaging the cells without the use of an optical dye or stain requires a special kind of optical imaging. The most common method to do this is using imaging techniques which encode refractive optical phase changes introduced by the cells into detectable intensity changes. Imaging techniques which work on this premise include Zernike phase contrast, differential interference contrast (DIC) and Hoffman modulation contrast. For the purposes of this discussion we will refer to all of these as phase contrast imaging.

Phase contrast imaging techniques rely on placing the cells on very high optical quality, optically clear substrate, mostly typically glass slides or thin plastic. The substrate must be optically transparent. In addition, the substrate must also be very flat, smooth and free of composition characteristics (i.e. air bubbles, material inhomogeneity from molding stresses, etc.) which, in-turn can cause refractive phase changes. Such phase changes result in imaging artifacts which can easily occlude the subtle phase variations introduced by the biological cells. Note, phase contrast micrscopy is very much distinct from "brightfield" microscopy. Brightfield imaging relies on light absorption by the specimen to encode intensity variations which are visible to the user or detection apparatus. Phase contrast imaging relies optical phase changes created by the specimen, and, as such can produce an image even if the specimen is mostly transparent as is the case with unlabeled biological cells.

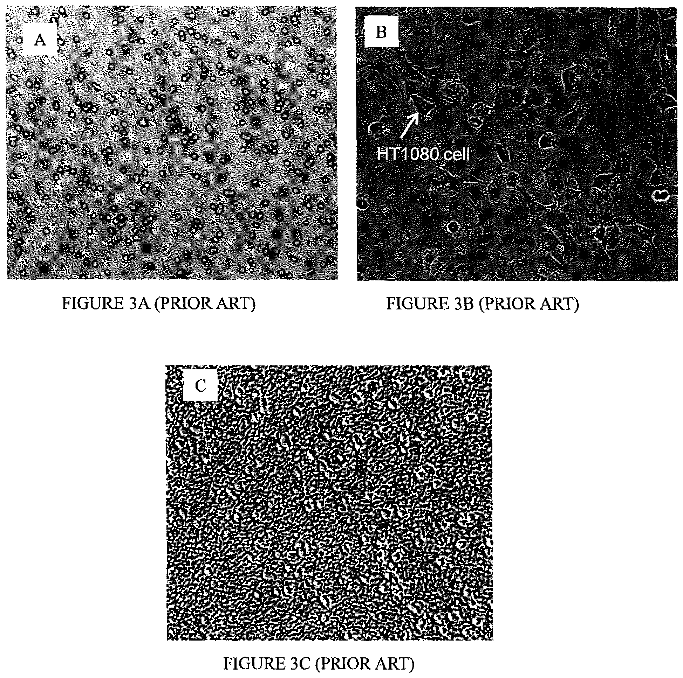

Phase contrast imaging is not amenable to the current commercially-available Boyden chamber consumables (prior art) which rely on porous membranes manufactured by the track-etch manufacturing process. Membranes produced by the track-etch process are not smooth, and introduce a variety of optical phase perturbations when imaged with a phase contrast microscope, making detection of the cells on the surface using these techniques impossible. Figure 3A, similar to Figure 2, is a brightfield image of a typical track-etch membrane used for biological studies. In this case the membrane has randomly spaced 8 micron diameter pores which are clearly visible under brightfield imaging.

Figure 3A is a brightfield image of a track-etch membrane with cells. Pores are visible and exhibit a random pattern under brightfield imaging. The particular membrane shown in Figure 3A also had migrating cancer cells (HT1080) on the surface, although under brightfield imaging the cells are not visible as previously described. By comparison, Figure 3B is an image of the same HT1080 cells on a clear plastic substrate taken at the same resolution, showing what "unlabeled" cells look like using phase contrast imaging on a high quality imaging substrate. Lastly, Figure 3C is a phase contrast image of the same track-etch membrane with HT1080 cells as shown in Figure 3A. As demonstrated by these series of images, the surface irregularities on the track-etch membrane surface result in an incomprehensible phase contrast image. Indeed, due to the optical perturbations caused by the membrane fabrication process, cells on the surface of the membrane cannot be identified.

The imaging artifacts introduced by these surface and material irregularities overwhelm the variations introduced by the cells making identification and enumeration of the cell number on these membranes using phase contrast imaging techniques impossible. As such, imaging of the cells on the surface of the existing track-etch membranes necessarily requires a fluorescent or optical dye to label (identify) the cells.

The practical impact of requiring an optical dye or fluorescent label to effectively "count" the cells is a big disadvantage in running these types of assays. First and foremost, many cell types are adversely affected by the presence of fluorescent or optical labeling dyes. It is well known that the presence of optical dyes can change cell viability, growth, and function. This is especially problematic when using "primary" hematopoetic blood cells (e.g. T-lymphocytes, neutrophils).

In addition, fluorescent or optical dye labeling techniques makes the assays more cumbersome and costly to perform. Lastly, the use of fluorescent or optical dyes often precludes the ability to take multiple time point recordings due to inherent phototoxicity and the generation of free radicals during the imaging process. One of the advantages of the techniques presented by the invention disclosed here is the ability to make these types of measurements on a substrate which is compatible with phase contrast imaging, thereby eliminating the requirement of labeling cells.

### B. Current Boyden Chamber devices are manufactured with porous membranes with no precise control of hole spacing or hole density

Boyden chambers rely on passive chemical diffusion in order for a gradient to be formed between the upper and lower fluid reservoirs. The chemical gradient formed is a complicated function of the pore geometry (hole size and spacing), time, concentration of the chemoattractant and the molecular weight of the chemoattractant. The gradient is, by definition, time varying and eventually, given enough time, the top chamber and bottom chamber concentrations equalize and the diffusion-based chemical gradient decays. While the geometry of track-etch membranes have been optimized for particle filtration, pore density/spacing using these membranes has not been optimized for measuring cell migration.

This situation is made worse by the non-homogeneous, random pore spacing of the track etch membranes (prior art) which causes time-dependent and local varying chemotactic gradients. In regions with high pore density, the chemical gradient can decay much quicker than in regions with low pore density. As the chemical gradient decays, the cells lose the ability to find the pores. These in turn can cause artifacts where, depending on the time of the experiment and concentration of the chemoattractant, the cells get confused and stop their directional migration. When this occurs, it becomes impossible to determine if a reduction in cell migration is due to degradation of the chemical gradient, or due to the inhibitory effect of a pharmacological test agent.

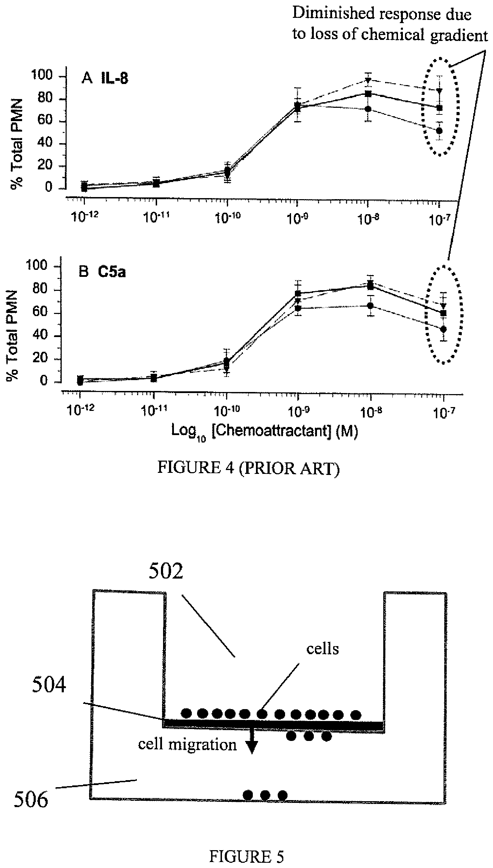

Figure 4 demonstrates an example of this effect using commercially available prior art Boyden chamber devices; in particular, the migration of human neutrophils in response to increasing concentrations of chemotactic agent in commercially available Boyden chamber measurements. Shown is the number of cells which have migrated through the track-etch membrane (vertical axis) vs. increasing concentrations of chemoattractant, IL-S in Figure 4A, and compound C5a in Figure 4B, respectively. As shown, at higher concentrations of chemoattractant, the cells undergo a diminished migratory response (dashed circle). This effect is inseparable from a diminished migratory response produced from an inhibitory compound and is generally the result of a time-concentration dependent decay of the chemotactic gradient. The practical consequence of this effect is that absolute comparisons between wells becomes a concentration and time dependent phenomenon requiring extreme diligence in experimental technique, adding imprecision to the resulting measurements and typically requiring the use of many replicate measurement wells per test compound to achieve assay precision.

### C. Number of cells required to quantify the response:

Another disadvantage of currently available Boyden chamber derived chemotaxis kits is that they require large number of cells to characterize the response, typically 50,000 to 100,000 cells per well [5] [6]. Large cell numbers can be a major cost/adoption disadvantage. This is especially the case when using rare, and perhaps difficult to isolate primary hematopoietic cells from the blood.

In summary, the Boyden chamber geometry remains the gold-standard measurement technique for measuring in-vitro chemotaxis. However, the commercial solutions (prior art) for the Boyden chamber geometry all suffer from the following disadvantages:
1.) Current Boyden chamber products manufactured with track-etch membranes are not amenable to phase contrast imaging, and therefore require that the cells be labeled for detection
2.) Fluorescent or optical dye staining of the cells can be detrimental to the physiology of the cells
3.) Current Boyden chamber products requires many cells per measurement well (typically 50,000 to 100,000)
4.) Current Boyden chamber products incorporating track-etch membranes have very non-uniform, irregular hole patterns which have not been optimized for this process. This results in non-homogeneous local chemical gradients at the cell surface, and local hole densities which are either too high (often) or too low. Combined, these characteristics tend to enhance the time-dependence of the gradient process, often making it difficult to distinguish a real pharmacological inhibition of migration from a time dependent decay in gradient.

### SUMMARY OF THE INVENTION

In accordance with the invention, a biological measurement apparatus according to claim 1 and a biological measurement method according to claim 10 are provided. Thereby, cells can be directly imaged and analyzed *in situ,* using phase contrast imaging techniques and *without* using fluorescent labels or optical dye staining.

Apparatus constructed in accordance with the invention includes a bottom reservoir, a top reservoir, and a thin porous optically transparent membrane having a top surface and a bottom surface separating the top and bottom reservoirs. In accordance with the invention, the pores of the membrane are manufactured using a laser-based photo-machining (ablation) process so that the porous optically transparent membrane has smooth surfaces free from aberrations enabling quantitative phase-contrast optical imaging of biological cells on the top or the bottom surface of the membrane without the use of fluorescent probes or optical staining. The hole diameter, spacing and density of the pores in the porous membrane can be precisely controlled, enhancing the applicability of these devices for chemotaxis and chemo-invasion type assays. For example, the pores formed in the membrane may have diameters in the range of 1 to 30 microns and spaced apart at a distance ranging from 5 to 500 microns. The pores may be arranged in a predetermined array having a rectangular or other geometry. The combined attributes of this invention result in greater assay precision using fewer cells.

The membrane may be composed of polyethylene terephthalate (PET), biaxially-oriented polyethylene terephthalate (boPET), polycarbonate, polyimide, polyether ether ketone (PEEK), polystyrene or other appropriate material with optical characteristics which do not introduce significant phase perturbation to an incoming light wavefront in relation to those introduced by biological cells on the surface of the membrane. In accordance with the invention, the membrane is substantially smooth and optically transparent even following pore formation, enabling quantitative phase-contrast optical imaging of biological cells. A plurality of upper and lower reservoirs may be provided to form a multi-well plate.

The invention may be used for the measurement of cell migration (chemotaxis), cell invasion, cell permeability, tissue remodeling, cell polarity endocrine signaling or cell transport. In a typical application, the porous membrane is used to separate upper and lower fluid-containing reservoirs and coated with collagen 1, fibronectin, laminin or other extracellular matrix material. An inverted phase contrast, DIC, or Hoffman Modulation-type microscope may be used to assess the morphology and/or number of biological cells on the membrane. An advantage of the invention is that chemotaxis, cell migration, cell invasion and other processes may be carried out by counting the cells on the surface of the porous membrane directly and without using fluorescent labels or optical dye staining.

The improvements made possible by the invention impact a range of potential biological applications where Boyden chamber geometries are currently used including co-culture studies, tissue remodeling studies, cell polarity determinations, endocrine signaling, cell transport, cell permeability, invasion and chemotaxis assays. While the description presented here is intended to describe the advantages of the invention specifically as it relates to the measurement of *in vitro* chemotaxis and *in vitro* cell invasion assays, many of the attributes associated with the invention are extendable to other common uses of Boyden chamber systems.

### BRIEF DESCRIPTION OF THE DRAWINGS

FIGURE 1 depicts a prior art single well of an existing Boyden chemotaxis chamber geometry;
FIGURE 2 shows a prior-art bright field image of a track-etch membrane surface;
FIGURES 3A-3C illustrate prior art brightfield images of a track-etch membrane with cells, a phase contrast image of cells on high quality plastic, and a phase contrast image of a track-etch membrane with cells;
FIGURE 4 illustrates a prior art artifact associated with a time, concentration dependent gradient degradation found in existing Boyden chamber chemotaxis assays;
FIGURE 5 illustrates a preferred embodiment of a Boyden chamber using a photo-machined membrane of the present invention;
FIGURE 6 shows a phase contrast image of a photomachined membrane demonstrating precise pore size and pore location. Also shown are HT1080 cells on the surface of the membrane;
FIGURE 7 shows time lapse phase contrast images demonstrating chemotactic directed migration of HT1080 cells on a photomachined membrane;
FIGURE 8 shows an embodiment of the present invention, a rectangular array of micro-holes forming the porous membrane of a Boyden chamber geometry;
FIGURES 9A-9B depict a preferred embodiment of the invention including a 96-well consumable comprised of 96 individual Boyden chambers using a laser photo-machined membrane; and
FIGURE 10 shows how an automated phase contrast imaging system can be used to image the cells on the surface of the membrane.

### DETAILED DESCRIPTION OF THE INVENTION

Traditional track-etch membranes used in the production of commercially available Boyden chamber devices use thin polymer films such as polycarbonate or polyethylene terephthalate (PET). The process of making pores in track-etch membranes involves bombarding the surface with radiation, typically alpha particles followed by a chemical etching step using highly concentrated NaOH or HF acid. The pore density (number of pores and density) is grossly controlled by the physical proximity and exposure levels to the radiation source. The pore size is determined by the concentration and exposure time in the etch step. The spatial location of the resulting etched pores is random as determined by the random particle generation of the radioactive source. The result of this process is a material that has significant optical surface blemishes. These blemishes prohibit the use of common non-labeled, non-invasive imaging techniques such as phase contrast, or differential interference contrast (DIC) to view cells *in situ* on the surface of the membranes.

According to this invention the porous membrane of the biological measurement apparatus is manufactured by a different manufacturing process, one based on laser-based photo-machining. Laser-based photo-machining generally uses a pulsed laser of the proper wavelength and pulse energy such that the optical energy is absorbed by the target material (in this case a thin polymer film) resulting in small pieces being ablated from the surface with each pulse. The laser beam can be precisely positioned in X-Y lateral dimensions and the beam dimension, optical power and pulse repetition frequency can be precisely controlled so as to allow for very fine micro-hole machining.

An objective of the invention is to provide a regular-spaced grid of micro-holes in a thin film whereby the pore density and pore size are carefully controlled and the resulting porous material has much improved optical surface characteristics in comparison to track-etch membranes (prior art). It should also be noted that there are many techniques for making such photomachined holes, including a range of laser excitation wavelengths, optical scanning systems and one, or two-dimensional laser mask approaches. All such techniques would be well known to those skilled in the art of laser photomachining processes and the specific implementation should not limit the invention presented here.

Figure 5 illustrates a specific embodiment of the invention, that being a Boyden Chamber single-well geometry having an upper chamber 502, a lower chamber 506, and a laser-photo machined membrane 504. In this preferred embodiment the substantially smooth, optically clear, laser photomachined porous membrane 504 replaces the track-etch membrane used in current commercial devices. The membrane in this case is of a thin transparent material which is sufficiently smooth to facilitate phase-contrast imaging techniques.

It is envisaged that much like current track-etch membranes, a variety of micro-hole sizes and holes spacings could be utilized to optimize measurement geometry for a particular cell type and assay. One of the advantages of this approach is being able to control the precise location and density of the photo-machined micro-holes. This is in contrast to the random location of the microholes as provided with prior-art track-etch membranes.

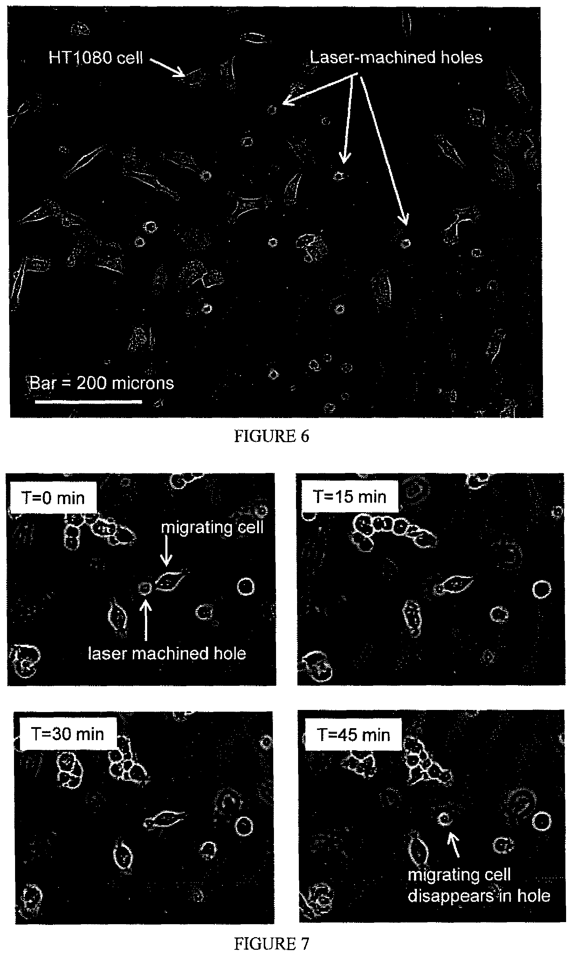

Figure 6 is a phase-contrast image of a photomachined membrane used in a Boyden chamber device constructed in our laboratory. In this example, the laser machined holes (9 total) were each drilled 8 microns in diameter and 180 microns apart in a regular 3 x 3 pattern. Also shown are HTI080 cells on the surface of the membrane. As shown by this example the use of a photomachined membrane allows for a.) imaging of the cells on the surface of the membrane using phase contrast imaging i.e. no labels and b.) precise hole size and hole locations to be constructed. Figure 6 very clearly shows the ability to detect the unlabeled biological cells on the surface of the membrane using phase contrast imaging. This image should be viewed in comparison with that of Figure 3C (prior-art) where the cells were not discernble using phase contrast imaging techniques on traditional track-etch membranes.

Figure 7 depicts a time lapse history of demonstrated cell chemotaxis of HT1080 cells on a photomachined membrane reduced to practice in our laboratory. In this example, the HT1080 cells were serum starved for 24 hours, and then loaded on the top side of the membrane (upper reservoir of the Boyden chamber) in serum free media. After the cells settled onto the membrane, the bottom chamber was then loaded with media and 10% serum. Once the device is fully primed, the serum diffuses (from high concentration to low) up through the individual microholes to the upper reservoir, thereby creating a local chemical gradient around each pore for the cells to follow. Once the cells reach the pore, they typically crawl through the hole, either to the bottom side of the membrane, or falling to the lower reservoir.

Using photo-machining to make the individual micro-holes provides, for the first time, the ability to control the spacing of the microholes. This helps alleviate several of the disadvantages of the current Boyden chamber systems previously described. First, by controlling the microhole spacing one can insure that the chemical gradient is spatially uniform and consistent around the microholes.
This is in comparison to the non-uniform local gradients resulting from random microhole patterns in the prior-art track-etch membranes.

Second, the holes can be precisely and regularly spaced such that all of the cells in the local vicinity of the pores will migrate (under optimum chemotactic conditions) to the holes. This, effectively, optimizes the measurement geometry by minimizing the number of holes required for a given cell density. Third, using a uniform pore spacing and minimizing the number of holes required extends and optimizes the time before the gradient starts to decay. This helps to eliminate the "roll-over" effect shown in Figure 4 by allowing all cells in the vicinity of the microholes to migrate before the gradient effectively decays.

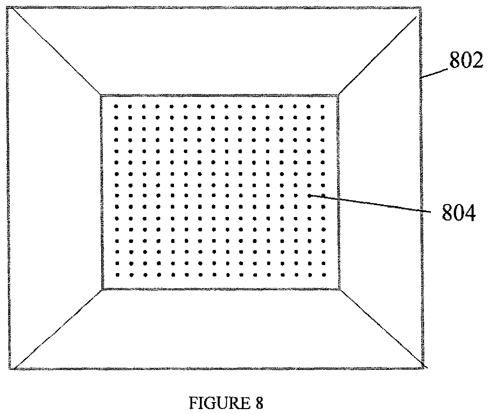

Figure 8 is a top view of a single well 802 depicting a preferred embodiment using a 16 x 16 array of photomachined microholes 804, 8 uM in diameter and 180 microns apart. The specific dimensions shown here are only examples, demonstrating the fact that, unlike the track-etch membranes, the pore spacing can be carefully controlled. Using this approach, one could design and implement many defined variations of micro-hole size and spatial density depending on the biological system under study. Variables would include optimizing the pore size for a given cell type, as well as the pore spacing for given experimental paradigm.

The use of laser photo-machining (ablation) to fabricate the porous membrane has additional advantages for biological applications. First, the photo-machining process is not detrimental to the optical quality of the thin film polymer material. Unlike the track-etch membrane process which requires a chemical etch of the entire surface to form the pores, the laser machining process has minimal detrimental impact on the optical quality of the film in the regions between the micro-holes as shown in Figures 6 and 7. Maintaining a high optical quality substrate enables quantitative, high contrast morphological analysis and/or cell counting of individual living cells on the membrane without using fluorescent labels or optical dyes. This is a big advantage over current Boyden chamber methodology which requires fluorescent labeling to image and count the cells due to the poor optical characteristics of the track-etch membranes.

A non-labeled approach is also more amenable to a kinetic, multiple time point read-out. Pre-labeling the cells before the experiment can lead to phototoxicity effects during the experiment. To avoid this, researchers often rely on "post labeling" the cells after the experiment has been performed. This latter approach, however, is by definition a single, end-point determination.

Having the ability to directly view the cells during the chemotaxis process allows researchers to study morphological changes, and or associate the response to other imaging parameters for example the "shape change" associated with a migrating or invasive morphological phenotype. One can not underestimate the value of being able to image the chemotactic process in real time when validating and/or interpreting assay data. It should also be understood that while the present invention does not require the use of fluorescent labels for detection, it does not preclude their use either. Aside from the improved compatibility with phase contrast imaging, better optical quality of the membrane will also improve the image quality when fluorescence detection is desired, such as may be necessary for analyzing mixed cell populations.

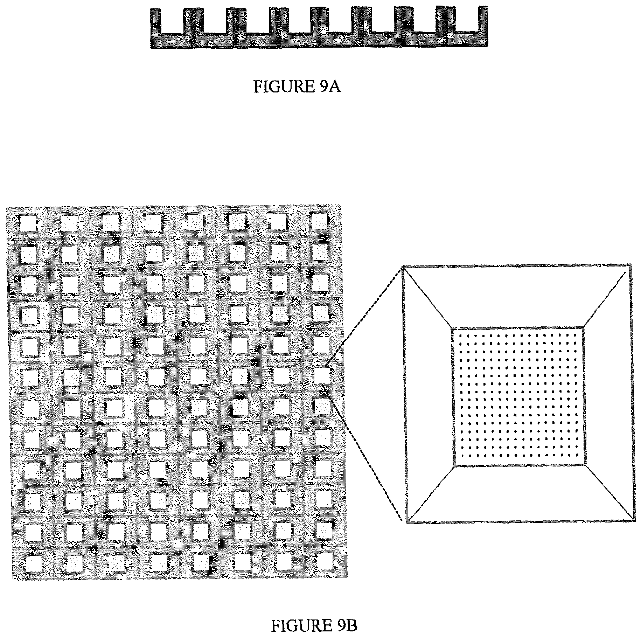

It should be noted, that the single-well geometry described in Figures 5 (preferred embodiment) can easily be extended to a rectangular array comprised of a plurality of wells and common formats used the biological sciences for example 6-well, 24-well, 96-well, 384-well and 1536-well geometries. An example of a 96-well format is depicted in Figures 9A (side view) and 9B (top view). Each of the individual wells would have its only two-dimensional grid of laser machined micropores. The commercial solutions previously described are generally found in 6-well, 24-well and 96-well formats.

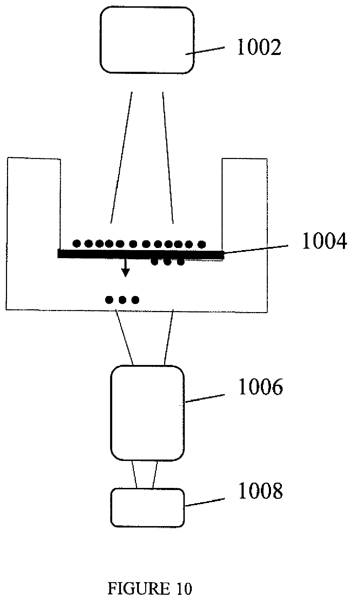

One of the anticipated benefits of this invention is to be able utilize the optimized geometry and enhanced precision in or to reduce the number of cells required per measurement chamber. We estimate being able to obtain improved data precision to existing Boyden chamber products using 1,000 to 5,000 cells per well, as opposed to the 50,000 to 100,000 cells required for existing commercially available Boyden chamber products. Figure 10 illustrates the automated imaging of a single-well of a Boyden chamber geometry using an optically clear, photo-machined membrane 1004. A light source supported above the membrane is shown at 1002, microscope objective disposed below the membrane is shown at 1006, and a detector (i.e., CCD camera) is depicted at 1008.

Precise photo-machining of the thin file polymer membrane 1004 leaves an optically smooth surface free from aberrations and enables the use of phase contrast, or other non-labeled, imaging techniques for enumerating the cells on the membrane in situ using manual or automated microscopy. This simple example of an automated phase contrast imaging geometry could be used to analyze all of the wells of a microplate-based consumable. Such systems are commercially available (e.g. Essen Biosciences' IncuCyte) and could be used to automatically focus on the membrane and quantify cell migration parameters in real time.

Although the invention described in this document is directed to a test chamber for measuring the migration of cells to chemical stimuli, e.g., chemotaxis or chemokinesis, the invention has applications beyond cell migration for example in the measurement of cell permeability, cell transport, cell invasion (others) where direct imaging of the surface would allow for non-invasive, quantitative assessment of the cells on the membrane *in situ.*

### ALTERNATIVE EMBODIMENTS

Different types of polymers and polymer thicknesses, amenable to the laser ablation photo-machining may be used. Variations in pore size, pore density and pore location are also anticipated. It is anticipated that pore geometry and spacing will be used to enhance the gradient homogeneity at the top surface of the membrane for various experimental paradigms. Various imaging systems could be used to image the membrane *in situ,* including epifluorescence (when fluorescent labeling is desired for other reasons), Zernike phase contrast, differential interference contrast (DIC), Hoffman modulation contrast and others. Different data processing schemes could also be utilized including measuring just the cells on the top side of the membrane, or alternatively measuring cells at three different planes being the a) top side of the membrane, b) bottom side of the membrane and at the bottom of the collection reservoir (lower chamber).

In order to reduce cell usage, many different potential reservoir configurations are possible, including moving to smaller reservoir formats, or smaller microplate formats such as a half-area 96-well format, 384-well format, or 1536-well format. Another alternative embodiment would be to apply biological coatings to the surface of the membrane such as collagen 1, fibronectin, or laminin. This type of coating would be a very thin molecular surface coating so as not to plug the microholes. It is also possible to apply a thicker extracellular matrix (ECM) coating to the membrane, where the Boyden chamber can be used to measure the ability of cells to invade the ECM, an assay known as a cell invasion assay. This is a natural extension and common use of existing Boyden chamber consumables.

In summary, in the biological measurement apparatus according to the invention the traditional track-etch membrane used in current Boyden chamber devices is replaced by a porous membrane manufactured via laser photomachining. There are several advantages of this invention over existing prior-art Boyden chamber devices. While much of this document has described the advantages for measurements of cell migration, many of these advantages are extendable to other applications for Boyden chambers with numerous practical benefits:
a) Laser-based photo-machining of the membrane allows precise control of both the pore size and pore spacing, enabling, for the first time, the ability to use these parameters to optimize the chemical gradient formation process when used as a chemotaxis or chemo-invasion measurement device. Minimizing the number of holes required, and providing the holes at defined locations provides a more uniform, temporally stable diffusional gradient in comparison to existing commercially available devices. It is anticipated that Boyden chamber devices constructed using this invention will provide more stable pharmacological data and reduce time dependent artifacts associated with random, non-optimized hole patterns found in existing devices (see Figure 4).
b) Replacing the track-etch membrane of the Boyden chamber with one manufactured by laser ablation allows, for the first time, imaging of the cells on the surface of the membrane *in situ* using non-labeled phase contrast imaging techniques. Consequently, cells no longer have to be exposed to potentially invasive, optical or fluorescent labeling dyes or protocols. This is very important for primary cells which can be time-sensitive, or label sensitive. It also save operator time and reduces reagent cost.
c) Direct imaging of the cells on the surface of the membrane, without using fluorescent probes or optical stains greatly enhances the extension of these assays to a homogeneous, automated, multi-time point data collection and analysis. Morphological changes to the cells, such as shape change can be monitored *in situ.* Direct non-labeled imaging of the cells during the migration processes can be used to help validate and interpret data.
d) Existing commercially available Boyden chamber solutions dictate that at least 50,000 to 100,000 cells are required per measurement well to achieve reasonable measurement precision. Due to the combined benefits of this invention (optimized pore spacing, in situ surface imaging), we anticipate needing only 1,000 to 5,000 cells per well to achieve comparable data precision to existing devices.

### REFERENCES

1.) Stephen Boyden, Ph.D., "The Chemotactic Effect of Mixtures of Antibody and Antigen on Polymorphonuclear Leucocytes" J. Exp. Med. 115: pp. 453-466, (1962).
2.) C.W. Frevert, V.A. Wong, R.B. Goodman, R. Goodwin, T.R. Martin, "Rapid fluorescence-based measurement of neutrophil migration in vitro, Journal of Immunological Methods 213 (1998) 41-52*.*
3.) J.A. Quinn, J.L. Ajnderson, W.S. Ho, and W.J. Petzny, J., Model Pores of Molecular Dimension, the Preparation and Characterization of Track-Etched Membranes, Biophysical Journal Volume 12, (1972)
4.) B. Heit, P. Colarusso, P. Kubes, "Fundamentally different roles for LFA-1, Mac-1 and alpha4-integrin in neutrophil chemotaxis, Journal of Cell Science 118 (22), (2005) 5205-5220.
5.) Corning Life Sciences Inc., Corning NY, *Cell Migration, Chemotaxis and Invasion Assay Protocol* - *CLS-AN-061*
6.) Suparna Sanyal, Susan Qian, Jeff Partridge and Marhsall Kosovsky, "Optimized Chemotaxis Conditions for Primary Blood Monocytes or THP-1 Cells using BD Falcon™ FluoroBlok™ 96-Multiwell Insert Plates, *Technical Bulletin #457, BD Biosciences,,* BD Biosciences-Discovery Labware, Bedford, MA 01730

## Claims

1. Biological measurement apparatus, comprising:
a bottom reservoir;
a top reservoir;
a thin porous optically transparent membrane having a top surface and a bottom surface separating the top and bottom reservoirs; and wherein:
the pores of the membrane are formed using a laser-based photo-machining(ablation) process; so that
the porous optically transparent membrane has smooth surfaces free from aberrations enabling quantitative phase-contrast optical imaging of biological cells on the top or the bottom surface of the membrane without the use of fluorescent probes or optical staining.

2. The apparatus of claim 1, wherein the membrane is composed of polyethyleneterephthalate (PET), biaxially-oriented polyethylene terephthalate (boPET), polycarbonate, polyimide, polyether ether ketone (PEEK), or polystyrene.

3. The apparatus of claim 1, where the porous membrane is in the range of 10 to 125 microns thick; or where the pores of the member are arranged in a predetermined rectangular or other geometric array;
or wherein the pores of the member have a uniform and consistent spacing, density and diameter; preferably wherein the pores of the membrane are spaced apart at a distance ranging from 10 to 500 microns;
or wherein the pores of the membrane have diameters in range of 1 to 15 microns.

4. The apparatus of claim 1, further including a plurality of upper and lower reservoirs forming a multi-well plate;
or wherein the membrane is coated with collagen 1, fibronectin, laminin or other extracellular matrix.

5. The apparatus of claim 1, wherein the reservoirs are manufactured with an injection molded plastic; or wherein the reservoirs are manufactured with injection molded polystyrene, polycarbonate, polyethylene terephthalate (PET) orbiaxially-oriented polyethylene terephthalate (boPET).

6. The apparatus of claim 1, wherein the membrane is attached to either the top or bottom reservoir using an ultrasonic welding process or chemical bonding agent; or wherein the membrane is attached to either the top or bottom reservoir using laser welding, or laser mask welding; or wherein the membrane is attached to the bottom surface of the top reservoir thereby forming an insert that fits inside the bottom reservoir.

7. The apparatus of claim 1, wherein the size of the reservoirs, the size of the pores, the number of the pores, and the location of the pores are optimized to reduce the number of biological cells needed for a given assay precision.

8. The apparatus of claim 1, wherein a plurality of upper reservoirs are attached to a porous membrane, thereby forming a removable insert that fits inside a plurality of co-aligned bottom reservoirs forming a microplate.

9. The apparatus of claim 1, further including a phase contrast imaging system for performing the quantitative optical imaging of biological cells on the membrane without the use of fluorescent probes or optical staining; preferably wherein the phase contrast imaging system utilizes one of Zernike phase contrast, differential interference contrast (DIC) or Hoffman modulation contrast.

10. A biological measurement method using the apparatus according to anyone of claims 1 - 9, comprising
performing quantitative phase-contrast optical imaging of biological cells on the upper surface of the membrane without the use of fluorescent probes or optical stains.

11. The method of claim 10, including the step of using a phase-contrast technique to perform the quantitative optical imaging; or including the step of using Zernike phase contrast, differential interference contrast (DIC), or Hoffman modulation contrast to perform the quantitative optical imaging.

12. The method of claim 10, further including the use of epifluorescence microscopy; or wherein the quantitative optical imaging is used for the measurement of cell migration (chemotaxis), cell invasion, cell permeability, tissue remodeling, cell polarity endocrine signaling or cell transport; or wherein the step of quantitative imaging involves a morphological assessment of shape, and/or the counting of the cells on the surface of the membrane and/or the identification of a particular cell type within a mixed cell population.

13. The method of claim 10, including the step of counting the number of cells remaining on the upper surface of the membrane over time to quantify cell chemotaxis or cell invasion; preferably including the use of kinetic, multi-time-point quantitative optical microscopic measurements to reduce artifacts associated witht ransient chemical gradients in chemotaxis or chemo-invasion assays.

## Patentansprüche

1. Biologische Messvorrichtung, die umfasst:
einen unteren Vorratsbehälter;
einen oberen Vorratsbehälter;
eine dünne poröse lichtdurchlässige Membran mit einer oberen Oberfläche und mit einer unteren Oberfläche, die den oberen und den unteren Vorratsbehälter trennt; und wobei:
die Poren der Membran unter Verwendung eines laserbasierten Photobearbeitungsprozesses (Ablationsprozesses) gebildet sind; sodass
die poröse lichtdurchlässige Membran glatte Oberflächen aufweist, die frei von Abweichungen sind, was eine quantitative optische Phasenkontrastbildgebung biologischer Zellen auf der oberen oder auf der unteren Oberfläche der Membran ohne Verwendung von Fluoreszenzsonden oder optische Färbung ermöglicht.

2. Vorrichtung gemäß Anspruch 1, wobei die Membran aus Polyethylenterephthalat (PET), biaxial orientiertem Polyethylenterephthalat (boPET), Polycarbonat, Polyimid, Polyetheretherketon (PEEK) oder Polystyrol besteht.

3. Vorrichtung gemäß Anspruch 1, wobei die poröse Membran im Bereich von 10 bis 125 Mikrometer dick ist; oder wobei die Poren des Elements in einer vorgegebenen rechteckigen oder anderen geometrischen Anordnung angeordnet sind;
oder wobei die Poren des Elements einen gleichförmigen und gleichbleibenden Abstand, eine gleichförmige und gleichbleibende Dichte und einen gleichförmigen und gleichbleibenden Durchmesser aufweisen; wobei die Poren der Membran vorzugsweise mit einem Abstand im Bereich von 10 bis 500 Mikrometer voneinander beabstandet sind;
oder wobei die Poren der Membran Durchmesser im Bereich von 1 bis 15 Mikrometer aufweisen.

4. Vorrichtung gemäß Anspruch 1, die ferner mehrere obere und untere Vorratsbehälter enthält, die eine Mehrmuldenplatte bilden;
oder wobei die Membran mit Kollagen 1, Fibronektin, Laminin oder einer anderen extrazellulären Matrix beschichtet ist.

5. Vorrichtung gemäß Anspruch 1, wobei die Vorratsbehälter mit einem Spritzgusskunststoff hergestellt sind; oder wobei die Vorratsbehälter mit Spritzgusspolystyrol, Spritzgusspolycarbonat, Spritzguss-Polyethylenterephthalat (Spitzguss-PET), orbiaxial orientiertem Polyethylenterephthalat (boPET) hergestellt sind.

6. Vorrichtung gemäß Anspruch 1, wobei die Membran unter Verwendung eines Ultraschallschweißprozesses oder eines chemischen Klebemittels entweder an dem oberen oder an dem unteren Vorratsbehälter befestigt ist; oder wobei die Membran unter Verwendung von Laserschweißen oder von Lasermaskenschweißen entweder an dem oberen oder an dem unteren Vorratsbehälter befestigt ist; oder wobei die Membran an der unteren Oberfläche des oberen Vorratsbehälters befestigt ist und dadurch einen Einsatz bildet, der in den unteren Vorratsbehälter passt.

7. Vorrichtung gemäß Anspruch 1, wobei die Größe der Vorratsbehälter, die Größe der Poren, die Anzahl der Poren und der Ort der Poren so optimiert sind, dass die Anzahl biologischer Zellen, die für eine gegebene Biotestgenauigkeit notwendig sind, verringert ist.

8. Vorrichtung gemäß Anspruch 1, wobei mehrere obere Vorratsbehälter an einer porösen Membran befestigt sind und dadurch einen abnehmbaren Einsatz bilden, der in mehrere gleichzeitig ausgerichtete untere Vorratsbehälter, die eine Mikroplatte bilden, passt.

9. Vorrichtung gemäß Anspruch 1, die ferner ein Phasenkontrastbildgebungssystem zum Ausführen der quantitativen optischen Bildgebung biologischer Zellen auf der Membran ohne Verwendung von Fluoreszenzsonden oder optischer Färbung enthält; wobei das Phasenkontrastbildgebungssystem vorzugsweise Zernike-Phasenkontrast oder Differential-Interferenzkontrast (DIC) oder Hoffman-Modulationskontrast nutzt.

10. Biologisches Messverfahren unter Verwendung der Vorrichtung nach einem der Ansprüche 1-9, wobei das Verfahren umfasst:
Ausführen einer quantitativen optischen Phasenkontrastbildgebung biologischer Zellen auf der oberen Oberfläche der Membran ohne Verwendung von Fluoreszenzsonden oder optischen Färbungen.

11. Verfahren gemäß Anspruch 10, das den Schritt des Verwendens einer Phasenkontrasttechnik zum Ausführen der quantitativen optischen Bildgebung enthält; oder das den Schritt des Verwendens des Zernike-Phasenkontrasts, des Differential-Interferenzkontrasts (DIC) oder des Hoffman-Modulationskontrasts zum Ausführen der quantitativen optischen Bildgebung enthält.

12. Verfahren gemäß Anspruch 10, das ferner die Verwendung der Auflichtfluoreszenzmikroskopie enthält; oder wobei die quantitative optische Bildgebung für die Messung der Zellwanderung (Zellchemotaxis), der Zellinvasion, der Zellpermeabilität, des Gewebeumbaus, der Zellpolaritätsendokrin-Signalisierung oder des Zelltransports verwendet wird; oder wobei der Schritt der quantitativen Bildgebung eine morphologische Beurteilung der Form und/oder das Zählen der Zellen auf der Oberfläche der Membran und/oder die Identifizierung eines bestimmten Zellentyps in einer gemischten Zellenpopulation umfasst.

13. Verfahren gemäß Anspruch 10, das den Schritt des Zählens der Anzahl der Zellen, die im Zeitverlauf auf der oberen Oberfläche der Membran verbleiben, um die Zellchemotaxis oder die Zellinvasion zu quantifizieren, enthält, wobei es vorzugsweise die Verwendung kinetischer quantitativer optischer mikroskopischer Mehrzeitpunktmessungen zum Verringern von Artefakten, die vorübergehenden chemischen Gradienten in der Chemotaxis oder in Chemoinvasions-Biotests zugeordnet sind, enthält.

## Revendications

1. Appareil pour mesures biologiques comprenant :
un réservoir inférieur ;
un réservoir supérieur ;
une membrane mince et poreuse optiquement transparente ayant une surface supérieure et une surface inférieure séparant les réservoirs supérieur et inférieur ; et dans lequel :
les pores de la membrane sont formés en utilisant un procédé de photo-usinage (ablation) au laser ; de sorte que
la membrane poreuse optiquement transparente présente des surfaces lisses exemptes de tout défaut, permettant d'effectuer l'imagerie optique à contraste de phase quantitative de cellules biologiques sur la surface supérieure ou inférieure de la membrane sans utiliser des sondes fluorescentes ou des colorants optiques.

2. Appareil de la revendication 1, dans lequel la membrane est composée de téréphtalate de polyéthylène (PET), téréphtalate de polyéthylène à orientation biaxiale (boPET), polycarbonate, polyimide, poly(éthercétone) (PEEK) ou polystyrène.

3. Appareil de la revendication 1, dans lequel l'épaisseur de la membrane poreuse est dans la plage de 10 à 125 microns ; ou dans lequel les pores de la membrane sont disposés selon un réseau rectangulaire ou un autre profil géométrique prédéterminé ;
ou dans lequel l'espacement, la densité et le diamètre des pores de la membrane sont uniformes et réguliers ; de préférence dans lequel les pores de la membrane sont séparés par une distance qui va de 10 à 500 microns ;
ou dans lequel les pores de la membrane ont des diamètres dans la plage de 1 à 15 microns.

4. Appareil de la revendication 1, qui comprend en outre une pluralité de réservoirs supérieurs et inférieurs formant une plaque à puits multiples ;
ou dans lequel la membrane est enduite de collagène de type I, de fibronectine, de laminine ou d'une autre matrice extracellulaire.

5. Appareil de la revendication 1, dans lequel les réservoirs sont constitués d'un plastique moulé par injection ; ou dans lequel les réservoirs sont constitués de polystyrène, polycarbonate, téréphtalate de polyéthylène (PET) ou téréphtalate de polyéthylène à orientation biaxiale (boPET) moulé par injection.

6. Appareil de la revendication 1, dans lequel la membrane est rattachée au réservoir supérieur ou inférieur en utilisant un processus de soudage aux ultrasons ou un agent de liaison chimique ; ou dans lequel la membrane est rattachée au réservoir supérieur ou inférieur par soudage au laser ou soudage au laser avec masque ; ou dans lequel la membrane est rattachée à la surface inférieure du réservoir supérieur, formant ainsi un insert qui s'emboîte dans le réservoir inférieur.

7. Appareil de la revendication 1, dans lequel la taille des réservoirs, la taille des pores, le nombre de pores et l'emplacement des pores sont optimisés pour réduire le nombre de cellules biologiques requises pour une précision analytique donnée.

8. Appareil de la revendication 1, dans lequel une pluralité de réservoirs supérieurs sont rattachés à une membrane poreuse, formant ainsi un insert amovible qui s'emboîte dans une pluralité de réservoirs inférieurs co-alignés formant une microplaque.

9. Appareil de la revendication 1, qui comprend en outre un système d'imagerie à contraste de phase permettant d'effectuer l'imagerie optique à contraste de phase quantitative de cellules biologiques sur la membrane sans utiliser des sondes fluorescentes ou des colorants optiques ; de préférence dans lequel le système d'imagerie à contraste de phase utilise une des techniques suivantes : contraste de phase de Zernike, contraste interférentiel différentiel ou contraste de modulation de Hoffman.

10. Procédé de mesure biologique utilisant l'appareil selon l'une quelconque des revendications 1-9, qui comprend
la réalisation de l'imagerie optique à contraste de phase quantitative de cellules biologiques sur la surface supérieure de la membrane sans utiliser des sondes fluorescentes ou des colorants optiques.

11. Procédé de la revendication 10, qui comprend l'étape consistant à utiliser une technique à contraste de phase pour effectuer l'imagerie optique quantitative ; ou qui comprend l'étape consistant à utiliser une technique à contraste de phase de Zernike, à contraste interférentiel différentiel ou à contraste de modulation de Hoffman pour effectuer l'imagerie optique quantitative.

12. Procédé de la revendication 10, qui comprend en outre l'utilisation de la microscopie en épifluorescence ; ou dans lequel l'imagerie optique quantitative est utilisée pour mesurer la migration cellulaire (chimiotactisme), l'invasion cellulaire, la perméabilité cellulaire, le remodelage tissulaire, la polarité cellulaire, la signalisation endocrinienne ou le transport cellulaire ; ou dans lequel l'étape d'imagerie quantitative fait intervenir une évaluation morphologique de la forme et/ou le dénombrement des cellules sur la surface de la membrane et/ou l'identification d'un type particulier de cellules dans une population de cellules mixtes.

13. Procédé de la revendication 10, qui comprend l'étape consistant à compter le nombre de cellules demeurant sur la surface supérieure de la membrane en fonction du temps pour quantifier le chimiotactisme cellulaire ou l'invasion cellulaire ; incluant de préférence l'utilisation de mesures cinétiques quantitatives obtenues par microscopie optique à des jalons temporels multiples pour réduire les artefacts associés aux gradients chimiques transitoires lors des tests de chimiotactisme ou de chimio-invasion.
